# EUROPEAN PATENT SPECIFICATION

(11) **EP 3 929 087 B1**
(45) Date of publication and mention of the grant of the patent: **17.09.2025**
(21) Application number: 21180303.6
(22) Date of filing: 18.06.2021
(51) Int. Cl.: B64D 11/06, B60N 2/879, A61L 2/00, A61L 2/10, B64F 5/30, A61L 9/20, B64D 13/06, B60N 2/00, B64D 11/00

(54) **ULTRAVIOLET LIGHT SHIELD SYSTEM**
ULTRAVIOLETTLICHTABSCHIRMSYSTEM
SYSTÈME DE PROTECTION CONTRE LA LUMIÈRE ULTRAVIOLETTE

(30) Priority: 23.06.2020 US 202063043105 P; 05.05.2021 US 202117308200
(43) Date of publication of application: 29.12.2021
(73) Proprietor: The Boeing Company, Arlington, VA 22202 (US)
(72) Inventor: CHILDRESS, Jamie J., Chicago, 60606-1596 (US)
(74) Representative: Howson, Richard Giles Bentham

(56) References cited:
- EP-A1- 3 061 465
- US-A1- 2014 179 212
- US-A1- 2019 030 195

## Description

### FIELD

Embodiments of the subject disclosure generally relate to systems and methods that may be used to sanitize structures and air within enclosed structures, such as vehicle cabins.

### BACKGROUND

Vehicles such as commercial aircraft are used to transport passengers between various locations. Systems are currently being developed to disinfect or otherwise sanitize surfaces to kill or neutralize various harmful microbes or other pathogens. Typical methods of sanitizing surfaces within aircraft involve significant manual effort by one or more crew members. For example, some crew members may spray and wipe cleaning chemicals on surfaces within an internal cabin of the aircraft. Other crew members may slowly wave a wand that emits ultraviolet (UV) radiation on nearby surfaces of the internal cabin. The UV radiation can kill or neutralize some microbes or other pathogens if held at a certain proximity to a target surface for at least a designated amount of time.

Furthermore, many commercial vehicles such as aircraft have HEPA filters in the air conditioning system that are able to entrap microbes and pathogens. The HEPA filters receive and clean air exiting the cabin or about to enter the cabin. HEPA filters and frequent cleaning of the cabin between flights are some methods to ensure the health of the passengers and crew onboard the aircraft. Additional sanitizing methods could be used to supplement the HEPA filters and chemical cleanings.

US2019/030195A1 in an abstract states that "A cabin disinfection system for an aircraft, including: a sensing means (3, 11, 13) to sense the presence of personnel in one or more zones (16-19, 23-26, 28, 29, 30, 32), a predictive sensing means (3, 11, 13, 33) to sense the direction of motion of a person (31) and to predict whether that person will occupy a zone to be disinfected (18) during planned disinfection, one or more UVC radiation sources (4, 9) to disinfect the cabin, and a controller (33) to control operation of the UVC radiation sources (4, 9) dependent on sensing of personnel in that zone and the predictive sensing means (3, 11, 13, 33) predicting that a person (31) will occupy the zone (18) during the planned disinfection period.

### SUMMARY

The present invention is set out in the independent claims, with some optional features set out in the claims dependent thereto.

A need exists for a system and a method for prohibiting the spread of pathogens between passengers onboard a vehicle during a trip, such as between passengers in an internal cabin of an aircraft during a flight, without risking harm to the passengers.

### BRIEF DESCRIPTION OF THE DRAWINGS

Figure 1 illustrates a perspective front view of an aircraft, according to some examples of the subject disclosure.
Figure 2 illustrates a perspective view of a UV light shield system within a portion of an internal cabin of the aircraft according to an embodiment of the claims.
Figure 3 illustrates a passenger on a seat that incorporates the UV light shield system according to an embodiment of the claims.
Figure 4 illustrates a perspective view of an internal cabin of an aircraft including a UV light shield system according to some examples which are not encompassed by the wording of the claims.
Figure 5 is a schematic diagram of the UV light shield system according to some examples.

### DETAILED DESCRIPTION

The foregoing summary, as well as the following detailed description of certain embodiments will be better understood when read in conjunction with the appended drawings. As used herein, an element or step recited in the singular and preceded by the word "a" or "an" should be understood as not necessarily excluding the plural of the elements or steps. Further, references to "one embodiment" are not intended to be interpreted as excluding the existence of additional embodiments that also incorporate the recited features. Moreover, unless explicitly stated to the contrary, embodiments "comprising" or "having" an element or a plurality of elements having a particular condition can include additional elements not having that condition.

The subject disclosure provides a UV light shield system that shields occupants of seats from pathogens along one or both sides of each occupant. The system utilizes UV lamps particularly mounted, positioned, and controlled to generate a virtual shield or side curtain using UV light emitted by the UV lamps. The shield or side curtain is defined by a field of illumination of the UV light. The UV light may be filtered at a designated wavelength or narrow wavelength range that is safe for human tissue. For example, the designated wavelength may be 222 nm. The system provides safe and effective sanitization by integrating the UV lamps into locations on one or both sides of the passenger seat depending on the location of the seat. For example, a middle seat may have UV lamps on both sides, while a window seat farthest from an aisle may only have a UV lamp between the window seat and the adjacent seat. The UV lamps emit light into multiple areas on either side of the passenger to form a virtual shield of UV light. Some of the UV light may impinge on a passenger in the seat, but a majority of the light is directed into the space along one or both sides of the passenger in a region adjacent to the upper body and head of the passenger. Thus, the UV light essentially forms side curtains, similar to side curtain airbags or stalls. The placement and/or control of the UV lamps may avoid direct close-range illumination of the passenger, such as the passenger's face, without continuing to shield the passenger.

In one or more examples, the system may be controlled (e.g., by a passenger, crew member, automatically based on sensor data, etc.) to persistently emit UV light to continuously disinfect air, surfaces, and people. In some examples, UV lamps of the shield system encompassed by the subject-matter of the claims are mounted in or on the sides of upper seat portions of passenger seat structures. In some examples not encompassed by the wording of the claims, one or more of the UV lamps are mounted to the back of the seat in front of the passenger, instead of on the seat occupied by that passenger. An air fan may be mounted in the seat structure in close proximity to the UV lamp to maintain air flow across the UV lamp. The fan air is sanitized by the UV light emitted by the UV lamp, and would push non-sanitized air away from the passenger. A reflector may be integrated within the UV lamp or disposed outside of the seat structure to direct the UV light to form the side curtain or stall illumination shape. The reflector may be used to direct most of the UV light into non-occupied spaces on either side of the passenger, and a small amount of UV onto the passenger. The UV lamps may be electrically connected to the existing wiring in the passenger seats, such as wiring for headrest display devices. The UV lamps may receive power from an onboard power source, such as one or more generators. In addition, sensors can be used by the system to automatically adjust power and duty cycles of the UV lamps when a passenger is out of their seat and/or within direct close range of a UV lamp.

One or more technical effects of the UV light shield system disclosed herein includes the ability to kill or neutralize a high percentage of pathogens (e.g., bacteria, virus, and the like) in the air on one or both sides of a passenger's head when the passenger is seated in an internal cabin. Pathogens in the air may be killed or neutralized by the UV side curtains prior to reaching the breathing area of the passenger. The UV light shield system may be harmless to the passengers due to the UV light having a designated narrow wavelength or range of wavelengths that is safe to human tissue. Still, the UV light shield system may take steps to avoid subjecting passengers to direct, close-range (e.g., high dose) UV light by reflecting the UV light into spaces along the sides of seats (where passenger upper bodies and heads are typically absent) and/or by automatically reducing power to or deactivating UV lamps upon detecting a passenger directly in front of a UV lamp (e.g., within a designated threshold proximity of the UV lamp). For example, the system may temporarily deactivate a UV lamp if a person is detected staring directly into the lamp from a few inches away. The UV shield system disclosed herein can supplement other methods for reducing the spread of pathogens between people, such as the use of face masks and social distancing.

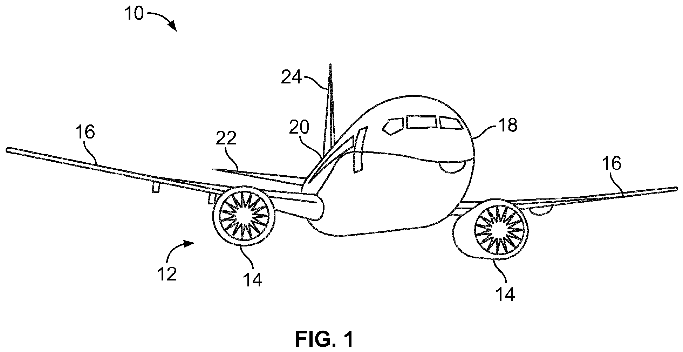

Figure 1 illustrates a perspective front view of an aircraft 10, according to some examples of the subject disclosure. The aircraft 10 includes a propulsion system 12 that includes engines 14, for example. Optionally, the propulsion system 12 may include more engines 14 than shown. The engines 14 are carried by wings 16 of the aircraft 10. In some examples, the engines 14 may be carried by a fuselage 18 and/or an empennage 20. The empennage 20 may also support horizontal stabilizers 22 and a vertical stabilizer 24.

The fuselage 18 of the aircraft 10 defines an internal cabin, which includes a flight deck or cockpit, one or more work sections (for example, galleys, personnel carry-on baggage areas, and the like), one or more passenger sections (for example, first class, business class, and coach sections), one or more lavatories, and/or the like.

Alternatively, instead of an aircraft, examples of the subject disclosure may be used with various other vehicles, such as automobiles, buses, rail vehicles, watercraft, and the like. For example, the UV light shield system disclosed herein can be implemented in an internal cabin of a passenger train, a bus, a passenger boat, and the like. Examples of the subject disclosure may also be used with respect to enclosed areas within fixed structures, such as commercial and residential buildings. For example, the shield system disclosed herein can be installed and operated within theatres, concert venues, places of worship, office buildings, hospitals, stores, auditoriums, classrooms, stadiums, and the like, where persistent UV light at non-harmful wavelengths can provide continuous disinfection of air and surfaces.

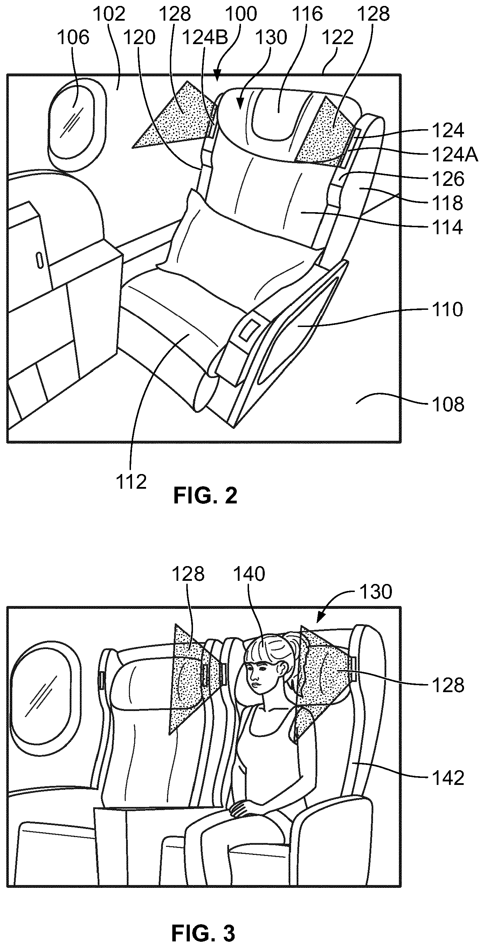

Figure 2 illustrates a perspective view of a UV light shield system 100 within a portion of an internal cabin 122 of the aircraft 10 according to an embodiment of the claims. The internal cabin 122 includes outboard walls 102 with windows 106 formed within the outboard walls 102. A floor 108 supports a plurality of passenger seats 110. The seat illustrated in Figure 2 may be a priority seat, such as a seat in a first-class section or a business section of the internal cabin 122. However, the system 100 disclosed herein can be used on seats in any section of the internal cabin 122, including coach.

The seat 110 has a cushion 112, a seat back 114, and a headrest cushion 116. The seat 110 has an inboard side 118 and an outboard side 120. The outboard side 120 faces towards the outboard wall 102. The UV light shield system 100 in the illustrated example includes two UV lamps 124 integrated into to the seat 110. The UV lamps 124 are light sources, such as excimer lamps, that emit light in the UV region of the electromagnetic spectrum. For example, a first UV lamp 124A is mounted to a front 126 of the seat back 114 at or proximate to the inboard side 118. The second UV lamp 124B is mounted to the front 126 of the seat back 114 at or proximate to the outboard side 120. The UV lamps 124 emit UV light in the forward direction. The direction and/or divergence of the UV light may be controlled such that the fields of illumination 128 constructively form side curtains or stalls of UV light. The field of illumination 128 of a UV lamp 124 refers to a three-dimensional volume in space that is defined by the propagation of UV light waves (e.g., rays) emitted by the UV lamp 124. The shape of the field of illumination 128 can depend on mechanical features of the UV lamp 124, such as reflectors, collimators, lenses, and the like, and in this case may be controlled to provide a shape that is tall in a vertical dimension and narrow in a lateral width dimension. The fields of illumination 128 may resemble panels or shields. The fields of illumination 128 bookend and define a passenger protection zone 130 therebetween. When a passenger is seated properly on the seat 110, the passenger is disposed within the passenger protection zone 130. The UV lamps 124 are mounted and positioned to direct UV light such that the fields of illumination 128 align generally with the upper body and head of the passenger.

Figure 3 illustrates a passenger 140 seated on a seat 142 that incorporates the UV light shield system 100 according to an embodiment of the claims. The passenger 140 is located in the protection zone 130. UV light in the fields of illumination 128 are disposed on both sides of the passenger 140. Some of the UV light may impinge upon the passenger's body, such as the shoulders, head, and arms, but a majority of the UV radiation is directed into the spaces to the side of the passenger 140. The UV light in the fields of illumination 128 may kill or neutralize pathogens in the air before the pathogens can be breathed by the passenger 140. The UV light shield system 100 can prevent the spread of at least some pathogens to and from the passenger 130 when the passenger 140 is in the seat 142.

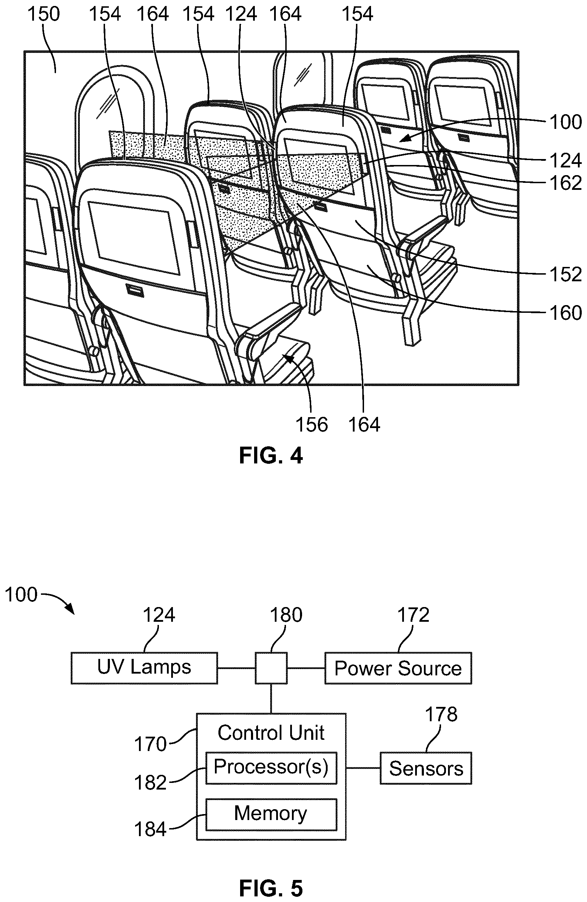

Figure 4 illustrates a perspective view of an internal cabin 150 of an aircraft including the UV light shield system 100 according to some examples not encompassed by the wording of the claims. In Figure 4, the UV lamps 124 are mounted along a rear 152 of a passenger seat 154. The UV lamps 124 are positioned to direct emitted UV light rearwards towards the seats 154 in a row 156 behind the seat 154 that holds the UV lamps 124. The UV lamps 124 are mounted along a seat back 160 of the seat 154, at or proximate to inboard and outboard sides 162, 164, respectively, of the seat 154. The UV light forms side curtains 164 or stalls for the seats 154 in the row 156 behind, extending between passengers seated in the row 156. Optionally, instead of mounting two UV lamps 124 to one seat 154 as shown in Figure 4, the outboard-most UV lamp 124 could be mounted instead to the adjacent seat 154 which is shown without any UV lamps 124.

In some examples, at least some UV lamps 124 may be mounted to a ceiling of the internal cabin above the seats 154 and positioned to direct UV light downwards (towards the floor) into the spaces along the sides of the seats 154 and passengers occupying the seats 154.

Figure 5 is a schematic diagram of the UV light shield system 100 according to an example (hereafter referred to as system 100). The system 100 the UV lamps 124, a control unit 170, sensors 178, and switching and/or conversion devices 180. UV lamps 124 are powered by an onboard power source 172 that supplies electrical power. The power source 172 may be a generator that converts mechanical energy to electrical energy. Various electrically conductive wires and cables may conduct the electrical power from the power source 172 to the UV lamps 124. For example, the UV lamps 124 may utilize the same conductive pathways that supply power to other components in the cabin, such as personal lights, headrest display units, and the like. For example, the UV lamps 124 may plug into the same electronics package that controls cabin lighting.

The control unit 170 is operatively connected to the UV lamps 124, the switching and/or power conversion devices 180, and the sensors 178 via wired and/or wireless communication pathways. The control unit 170 generates control signals that control the operations of the UV lamps 124. The control signals that are generated may be based on signals (e.g., data) received from the sensors 178. The control unit 170 represents hardware circuitry that includes and/or is connected with one or more processors 182 (e.g., one or more microprocessors, integrated circuits, microcontrollers, field programmable gate arrays, etc.). The control unit includes and/or is connected with a tangible and non-transitory computer-readable storage medium (e.g., memory) 184. For example, the memory 184 may store programmed instructions (e.g., software) that is executed by the one or more processors 182 to perform the operations of the control unit 170 described herein.

The control unit 170 can control the UV lamps 124 by controlling the presence and amount of electrical power (e.g., voltage and/or current) that is supplied to each of the UV lamps 124. For this function, the control unit 170 may utilize the switching and/or power conversion devices 180. The switching and/or power conversion devices 180 can include one or more solid-state relays, electromechanical relays, optical switches, power converters (e.g., DC-to-DC, DC-to-AC), and/or the like. The control unit 170 can deactivate or shut off one or more of the UV lamps 124 and modify a non-zero power output of one or more of the UV lamps 124 via the switching and/or power conversion devices 180. The switching and/or power conversion devices 180 may enable variable control over the amount of power supplied to the associated UV lamps 124, besides merely turning the lamps 124 ON (e.g., active and emitting UV light) and OFF (e.g., inactive and not emitting UV light). The variable power levels can include multiple settings, such as low, medium, and high.

In one or more examples, the UV light emitted by the UV lamps 124 is controlled to enable the occupants (e.g., passengers and crew) to be exposed to the UV light for a prolonged period of time without harm. For example, the emitted UV light may have a designated wavelength or a narrow band of wavelengths experimentally determined to be harmless to human tissue through prolonged exposure. Thus, even if the UV lamps 124 persistently emit UV light through the duration of the flight, the passengers would be unharmed. In a non-limiting example, the designated wavelength is 222 nm. It has been found that sanitizing UV light having a wavelength of 222 nm kills pathogens (such as viruses and bacteria), instead of inactivating pathogens. In contrast, UVC light at a wavelength of 254 nm inactivates pathogens by interfering with their DNA, resulting in temporary inactivation, but may not kill the pathogens. Instead, the pathogen may be reactivated by exposure to ordinary white light at a reactivation rate of about 10% per hour. As such, UVC light at a wavelength of 254 nm may be ineffective in illuminated areas, such as within an internal cabin of a vehicle. Moreover, UVC light at 254 nm is not recommended for human exposure because it may be able to penetrate human cells. In contrast, sanitizing UV light having a wavelength of 222 nm is safe for human exposure and kills pathogens. Further, the sanitizing UV light having a wavelength of 222 nm may be emitted at full power within one millisecond or less of the UV lamps 124 being activated (in contrast the UVC light having a wavelength of 254 nm, which may take seconds or even minutes to reach full power).

The sensors 178 may monitor the occupancy of the seats and/or the proximity of passengers relative to the UV lamps 124. For example, a first subset of sensors 178 may be configured to monitor the occupancy of seats, to determine whether each seat is occupied. The first subset can include pressure sensors, optical sensors, proximity sensors, cameras, or the like. A pressure sensor may be integrated into the cushion or seat back to register a change in the sensor signal responsive to a passenger sitting on and standing up from the seat. An optical sensor may detect when an optical beam proximate to the cushion of the seat is interrupted, which may occur as a person sits down and stands up from the seat. A proximity sensor mounted to the seat can utilize infrared and/or microwaves to determine when a person is present in the seat. The sensors 178 can generate sensor signals that are transmitted to the control unit 170 periodically at regular intervals or irregularly in response to detected monitoring variations, such as the optical beam being interrupted. The sensor signals can identify the source of the signals, such as the individual sensors that generate each of the signals. The control unit 170 receives the signals from the sensors 178 and analyzes the signals to determine the occupancy of each of the seats. In some examples, if a seat is determined to be vacant and unoccupied for at least a threshold amount of time (e.g., 1 minute), the control unit 170 is configured to reduce the power setting of the one or more UV lamps 124 that provide the UV side curtains for that seat. The power setting can be reduced to a low setting or the UV lamp(s) can be deactivated, at least until the sensor signals indicate that the seat is occupied. Once the control unit 170 determines that the passengers in two adjacent seats are both present in the seats, the UV lamp 124 that emits UV light between the two adjacent seats may be operated at high or full power to sanitize the air between the two passengers.

A second subset of sensors 178 may be configured to monitor proximity of the passengers to the UV lamps 124. The second subset can be proximity sensors that are mounted on or proximate to the UV lamps 124. The proximity sensors can use infrared or microwaves to determine when a person is within a designated threshold proximity to the UV lamp 124. The designated threshold proximity may be a few inches, such as 0.0508m (2 inches), 0.1016m (4 inches), 0.1524m (6 inches), 0.2032m (8 inches), or 0.254m (10 inches). The proximity sensors may be aimed to focus on proximity of objects within the field of illumination of the UV light, ignoring the proximity of a person's head that is next to the UV lamp 124 but not in the field of illumination. In response to receiving sensor signals that indicate that a person has crossed the threshold proximity, the control unit 170 may reduce the power setting of the associated UV lamp 124. The power setting can be reduced to a low setting or the UV lamp can be deactivated, at least until the sensor signals indicate that the seat is occupied.

Unless the proximity threshold is crossed or the seat is unoccupied, the control unit 170 may control the UV lamps 124 to persistently emit UV light during a trip of the vehicle to provide continuous protection from pathogens. Optionally, the control unit 170 may be configured to universally modulate the power settings of the UV lamps 124 based on various factors, such as a level of activity in the cabin, time of day, and the like. For example, the UV lamps 124 may be operated at high or full power when passengers are boarding and deboarding the vehicle. When the vehicle is traveling/cruising during the day, the control unit 170 may operate the UV lamps 124 at a medium power setting to conserve energy relative to the high power setting. When the vehicle is traveling/cruising during the night, the control unit 170 may operate the UV lamps 124 at a low power setting because most people are relatively inactive, either sleeping, reading, or watching videos. Furthermore, the intensity of the UV lighting can be increased for very rapid disinfection without excessive human exposure. The control operations followed by the control unit 170 may be stored in the memory 184.

The UV light shield system 100 disclosed herein may sanitize air coming into the seated passenger's space without shining the UV light directly onto the passenger. The UV lamps are placed to primarily provide protection to the face and upper body of the person being protected. Reflectors can be used direct most of the UV into non-occupied spaces on either side of the passenger and a small amount of UV onto the passenger themselves. The UV lamps can be placed, according to examples encompassed by the wording of the claims, in the upper seat sides or, according to examples not encompassed by the wording of the claims, in the seat back of the seat in front of the passenger. Sensors can be used to adjust UV power and duty cycle when a passenger is in direct close range of the UV lamp.

As described herein, examples of the subject disclosure provide systems and methods for sanitizing and disinfecting surfaces, air, and people within an internal cabin of a vehicle, particularly in passenger seats, using UV light without harming the people exposed to the UV light. Further, examples of the subject disclosure provide air sanitization shields formed via UV light. The UV shields are provided on one or both sides of a passenger in a seat, similar to side curtains or stalls, and sanitize air before the air reaches the passenger and/or before air from the passenger's zone reaches another person or surface.

While various spatial and directional terms, such as top, bottom, lower, mid, lateral, horizontal, vertical, front and the like can be used to describe examples of the subject disclosure, it is understood that such terms are merely used with respect to the orientations shown in the drawings. The orientations can be inverted, rotated, or otherwise changed, such that an upper portion is a lower portion, and vice versa, horizontal becomes vertical, and the like.

As used herein, a structure, limitation, or element that is "configured to" perform a task or operation is particularly structurally formed, constructed, or adapted in a manner corresponding to the task or operation. For purposes of clarity and the avoidance of doubt, an object that is merely capable of being modified to perform the task or operation is not "configured to" perform the task or operation as used herein.

As used herein, value modifiers such as "about," "substantially," and "approximately" inserted before a numerical value indicate that the value can represent other values within a designated threshold range above and/or below the specified value, such as values within 5%, 10%, or 15% of the specified value.

It is to be understood that the above description is intended to be illustrative, and not restrictive. For example, the above-described embodiments (and/or aspects thereof) can be used in combination with each other insofar as these fall within the scope of the claims. In addition, many modifications can be made to adapt a particular situation or material to the teachings of the various embodiments of the disclosure without departing from their scope. While the dimensions and types of materials described herein are intended to define the parameters of the various embodiments of the disclosure, the embodiments are by no means limiting and are exemplary embodiments. Many other embodiments will be apparent to those of skill in the art upon reviewing the above description. The scope of the various embodiments of the disclosure should, therefore, be determined with reference to the appended claims. In the appended claims and the detailed description herein, the terms "including" and "in which" are used as the plain-English equivalents of the respective terms "comprising" and "wherein." Moreover, the terms "first," "second," and "third," etc. are used merely as labels, and are not intended to impose numerical requirements on their objects.

## Claims

1. An ultraviolet UV light shield system (100) comprising:
a plurality of passenger seats configured to be disposed within an internal cabin of a vehicle, each passenger seat comprising a seat back (114) having a front (126);
multiple UV lamps (124) configured to be mounted within the internal cabin (122) and positioned to emit UV light in fields of illumination (128) that extend along sides (118, 120) of the passenger seats (110) wherein two adjacent fields of illumination (128) are spaced apart to define a protection zone (130) for a passenger (140) sitting on one of the passenger seats (110), the protection zone being along both sides of the one of the passenger seats; wherein two of the UV lamps (124) are mounted to the front (126) of the seat back (114) of the one passenger seat (110) such that one of the two UV lamps (124) is located at or proximate to the inboard side (118) of the one passenger seat (110) and the other of the two UV lamps (124) is located at or proximate to the outboard side (120) of the one passenger seat (110), the two UV lamps (124) provide the two adjacent fields of illumination (128).

2. The UV light shield system (100) of claims 1, wherein the two adjacent fields of illumination (128) align with an upper body and head of the passenger (140).

3. The UV light shield system (100) of any of claims 1-2, wherein the UV lamps (124) are configured to emit the UV light at a designated wavelength or narrow wavelength range that is safe for human tissue.

4. The UV light shield system (100) of claim 3, wherein the designated wavelength is 222 nm.

5. The UV light shield system (100) of any of claims 1-4, further comprising a control unit (170) and sensors (178), the control unit (170) including one or more processors (182) and operatively connected to the UV lamps (124) and the sensors (178), the sensors (178) configured to monitor the passenger seats (110), the control unit (170) configured to determine an occupancy of the passenger seats (110) based on sensor signals received from the sensors (178).

6. The UV light shield system (100) of claim 5, wherein responsive to determining that the passenger (140) in the one passenger seat (110) has unoccupied the one passenger seat (110) for at least a threshold period of time, the control unit (170) is configured to reduce a power setting of at least one of the UV lamps (124) that provide the two fields of illumination (128).

7. The UV light shield system (100) of claim 5 or 6, wherein the sensors (178) comprise at least one of pressure sensors (178), optical sensors (178), or proximity sensors (178).

8. The UV light shield system (100) of any of claims 1-4, further comprising a control unit (170) and sensors (178), the control unit (170) including one or more processors (182) and operatively connected to the UV lamps (124) and the sensors (178), the sensors (178) disposed on or proximate to the UV lamps (124), the control unit (170) configured to detect when persons are within a designated proximity threshold of the UV lamps (124) based on sensor signals received from the sensors (178); and optionally wherein, responsive to detecting that a person is within the designated proximity threshold of a first UV lamp of the UV lamps (124), the control unit (170) is configured to deactivate the first UV lamp or reduce a power setting of the first UV lamp without deactivating the first UV lamp.

9. The UV light shield system (100) of any of claims 1-8, wherein the vehicle is an aircraft (10).

10. A method for sanitizing to shield passengers in seats (110) disposed within an internal cabin of a vehicle, each passenger seat comprising a seat back (114) having a front (126), the method comprising:
mounting UV lamps (124) within the internal cabin (122) of the vehicle, the UV lamps (124) mounted to emit and direct UV light in fields of illumination (128) that extend along sides (118, 120) of the passenger seats (110) wherein two adjacent fields of illumination (128) are spaced apart to define a protection zone (130) for a passenger (140) sitting on one of the passenger seats (110), the protection zone being along both sides of one of the passenger seats; and
controlling the UV lamps (124) to emit the UV light in the fields of illumination (128) along both sides of the one of the passenger seats; wherein two of the UV lamps (124) are mounted to the front (126) of the seat back (114) of the one passenger seat (110) such that one of the two UV lamps (124) is located at or proximate to the inboard side (118) of the one passenger seat (110) and the other of the two UV lamps (124) is located at or proximate to the outboard side (120) of the one passenger seat (110), the two UV lamps (124) provide the two adjacent fields of illumination (128).

11. The method of claim 10, wherein the UV lamps (124) are controlled:
to shape the emission of UV light such that the fields of illumination (128) are elongated in a vertical dimension and narrow in a lateral dimension; and/or
such that the emitted UV light has a designated wavelength or narrow wavelength range that is safe for human tissue at prolonged exposure.

12. The method of claim 10 or 11, further comprising determining:
that one of the passenger seats (110) is unoccupied for at least a threshold period of time, and, in response, reducing a power setting of at least one of the UV lamps (124) that provide fields of illumination (128) that extend along the unoccupied passenger seat (110); and/or
that one of the passengers is within a designated proximity threshold of one of the UV lamps (124), and, in response, reducing a power setting of the one UV lamp.

13. An ultraviolet (UV) light shield system (100) comprising:
the UV light shield system (100) of any of claims 1-4;
sensors (178) configured to monitor occupancy of the passenger seats (110) and proximity of passengers to the UV lamps (124); and
a or the control unit (170) including one or more processors (182) and operatively connected to the UV lamps (124) and the sensors (178), the control unit (170) configured to receive sensor signals from the sensors (178) and modulate a power output of one or more of the UV lamps (124) based on the occupancy of the passenger seats (110) and the proximity of the passengers to the UV lamps (124).

## Patentansprüche

1. Ultraviolett- bzw. UV-Lichtabschirmsystem (100), umfassend:
eine Vielzahl von Passagiersitzen, die konfiguriert sind, in einer internen Kabine eines Fahrzeugs angeordnet zu sein, wobei jeder Passagiersitz eine Sitzlehne (114) mit einer Vorderseite (126) umfasst;
mehrere UV-Lampen (124), die konfiguriert sind, in der internen Kabine (122) montiert zu sein, und positioniert sind, um UV-Licht in Beleuchtungsfeldern (128) zu emittieren, die sich entlang Seiten (118, 120) der Passagiersitze (110) erstrecken, wobei zwei benachbarte Beleuchtungsfelder (128) beabstandet sind, um eine Schutzzone (130) für einen Passagier (140) zu definieren, der auf einem der Passagiersitze (110) sitzt, wobei die Schutzzone entlang beider Seiten des einen der Passagiersitze liegt; wobei zwei der UV-Lampen (124) an der Vorderseite (126) der Sitzlehne (114) des einen Passagiersitzes (110) montiert sind, sodass sich eine der beiden UV-Lampen (124) bei oder in der Nähe der Innenbordseite (118) des einen Passagiersitzes (110) befindet und sich die andere der beiden UV-Lampen (124) bei oder in der Nähe der Außenbordseite (120) des einen Passagiersitzes (110) befindet, wobei die beiden UV-Lampen (124) die beiden benachbarten Beleuchtungsfelder (128) bereitstellen.

2. UV-Lichtabschirmsystem (100) nach Anspruch 1, wobei die zwei benachbarten Beleuchtungsfelder (128) mit einem Oberkörper und dem Kopf des Passagiers (140) ausgerichtet sind.

3. UV-Lichtabschirmsystem (100) nach einem der Ansprüche 1-2, wobei die UV-Lampen (124) konfiguriert sind, um das UV-Licht mit einer designierten Wellenlänge oder einem schmalen Wellenlängenbereich zu emittieren, die bzw. der für menschliches Gewebe sicher ist.

4. UV-Lichtabschirmsystem (100) nach Anspruch 3, wobei die designierte Wellenlänge 222 nm beträgt.

5. UV-Lichtabschirmsystem (100) nach einem der Ansprüche 1-4, ferner umfassend eine Steuereinheit (170) und Sensoren (178), wobei die Steuereinheit (170) einen oder mehrere Prozessoren (182) beinhaltet und operativ mit den UV-Lampen (124) und den Sensoren (178) verbunden ist, wobei die Sensoren (178) konfiguriert sind, um die Passagiersitze (110) zu überwachen, wobei die Steuereinheit (170) konfiguriert ist, um eine Belegung der Passagiersitze (110) basierend auf Sensorsignalen zu bestimmen, die von den Sensoren (178) empfangen werden.

6. UV-Lichtabschirmsystem (100) nach Anspruch 5, wobei als Reaktion auf das Bestimmen, dass der Passagier (140) in dem einen Passagiersitz (110) den einen Passagiersitz (110) für zumindest einen Schwellenzeitraum belegt hat, die Steuereinheit (170) konfiguriert ist, um eine Leistungseinstellung mindestens einer der UV-Lampen (124) zu reduzieren, die die beiden Beleuchtungsfelder (128) bereitstellen.

7. UV-Lichtabschirmsystem (100) nach Anspruch 5 oder 6, wobei die Sensoren (178) mindestens einen von Drucksensoren (178), optischen Sensoren (178) oder Näherungssensoren (178) umfassen.

8. UV-Lichtabschirmsystem (100) nach einem der Ansprüche 1-4, ferner umfassend eine Steuereinheit (170) und Sensoren (178), wobei die Steuereinheit (170) einen oder mehrere Prozessoren (182) beinhaltet und operativ mit den UV-Lampen (124) und den Sensoren (178) verbunden ist, wobei die Sensoren (178) an oder in der Nähe der UV-Lampen (124) angeordnet sind, wobei die Steuereinheit (170) konfiguriert ist, um zu detektieren, wann sich Personen innerhalb einer designierten Näherungsschwelle der UV-Lampen (124) befinden, basierend auf Sensorsignalen, die von den Sensoren (178) empfangen werden; und optional wobei, als Reaktion auf das Detektieren, dass sich eine Person innerhalb der designierten Näherungsschwelle einer ersten UV-Lampe der UV-Lampen (124) befindet, die Steuereinheit (170) konfiguriert ist, um die erste UV-Lampe zu deaktivieren oder eine Leistungseinstellung der ersten UV-Lampe zu reduzieren, ohne die erste UV-Lampe zu deaktivieren.

9. UV-Lichtabschirmsystem (100) nach einem der Ansprüche 1-8, wobei das Fahrzeug ein Flugzeug (10) ist.

10. Verfahren zur Desinfektion zum Abschirmen von Passagieren auf Sitzen (110), die in einer internen Kabine eines Fahrzeugs angeordnet sind, wobei jeder Passagiersitz eine Sitzlehne (114) mit einer Vorderseite (126) umfasst, wobei das Verfahren umfasst:
Montieren von UV-Lampen (124) in der internen Kabine (122) des Fahrzeugs, wobei die UV-Lampen (124) montiert sind, um UV-Licht in Beleuchtungsfeldern (128) zu emittieren und zu lenken, die sich entlang Seiten (118, 120) der Passagiersitze (110) erstrecken, wobei zwei benachbarte Beleuchtungsfelder (128) beabstandet sind, um eine Schutzzone (130) für einen Passagier (140) zu definieren, der auf einem der Passagiersitze (110) sitzt, wobei die Schutzzone entlang beider Seiten eines der Passagiersitze liegt; und
Steuern der UV-Lampen (124), um das UV-Licht in den Beleuchtungsfeldern (128) entlang beider Seiten des einen der Passagiersitze zu emittieren; wobei zwei der UV-Lampen (124) an der Vorderseite (126) der Sitzlehne (114) des einen Passagiersitzes (110) montiert sind, sodass sich eine der beiden UV-Lampen (124) bei oder in der Nähe der Innenbordseite (118) des einen Passagiersitzes (110) befindet und sich die andere der beiden UV-Lampen (124) bei oder in der Nähe der Außenbordseite (120) des einen Passagiersitzes (110) befindet, wobei die beiden UV-Lampen (124) die beiden benachbarten Beleuchtungsfelder (128) bereitstellen.

11. Verfahren nach Anspruch 10, wobei die UV-Lampen (124) gesteuert werden:
um die Emission von UV-Licht zu steuern, sodass die Beleuchtungsfelder (128) in einer vertikalen Ausdehnung verlängert sind und in einer seitlichen Ausdehnung schmal sind; und/oder
sodass das emittierte UV-Licht eine designierte Wellenlänge oder einen schmalen Wellenlängenbereich aufweist, die bzw. der für menschliches Gewebe bei anhaltender Aussetzung sicher ist.

12. Verfahren nach Anspruch 10 oder 11, ferner umfassend Bestimmen:
dass einer der Passagiersitze (110) für zumindest einen Schwellenzeitraum unbelegt ist, und als Reaktion darauf, Reduzieren einer Leistungseinstellung mindestens einer der UV-Lampen (124), die Beleuchtungsfelder (128) bereitstellen, die sich entlang des unbelegten Passagiersitzes (110) erstrecken; und/oder
dass sich einer der Passagiere innerhalb einer designierten Näherungsschwelle von einer der UV-Lampen (124) befindet, und als Reaktion darauf, Reduzieren einer Leistungseinstellung der einen UV-Lampe.

13. Ultraviolett(UV)-Lichtabschirmsystem (100), umfassend:
das UV-Lichtabschirmsystem (100) nach einem der Ansprüche 1-4;
Sensoren (178), die konfiguriert sind, um die Belegung der Passagiersitze (110) und die Nähe von Passagieren zu den UV-Lampen (124) zu überwachen; und
eine oder die Steuereinheit (170), die einen oder mehrere Prozessoren (182) beinhaltet und operativ mit den UV-Lampen (124) und den Sensoren (178) verbunden ist, wobei die Steuereinheit (170) konfiguriert ist, um Sensorsignale von den Sensoren (178) zu empfangen und eine Leistungsausgabe einer oder mehrerer der UV-Lampen (124) basierend auf der Belegung der Passagiersitze (110) und der Nähe der Passagiere zu den UV-Lampen (124) zu modulieren.

## Revendications

1. Système de protection contre les rayons ultraviolets (100) comprenant :
une pluralité de sièges passagers configurés pour être disposés à l'intérieur d'une cabine interne d'un véhicule, chaque siège passager comprenant un dossier (114) ayant une partie avant (126) ;
plusieurs lampes UV (124) configurées pour être montées à l'intérieur de la cabine interne (122) et positionnées de manière à émettre une lumière UV dans des champs d'éclairage (128) qui s'étendent le long des côtés (118, 120) des sièges passagers (110), deux champs d'éclairage adjacents (128) étant espacés l'un de l'autre pour définir une zone de protection (130) pour un passager (140) assis sur l'un des sièges passagers (110), la zone de protection s'étendant le long des deux côtés dudit siège passager ; où deux des lampes UV (124) sont montées sur l'avant (126) du dossier (114) dudit siège passager (110) de telle sorte que l'une des deux lampes UV (124) soit située au niveau ou à proximité du côté intérieur (118) dudit siège passager (110) et l'autre des deux lampes UV (124) soit située au niveau ou à proximité du côté extérieur (120) du siège passager (110), les deux lampes UV (124) fournissant les deux champs d'éclairage adjacents (128).

2. Système de protection contre les rayons UV (100) selon la revendication 1, dans lequel les deux champs d'éclairage adjacents (128) sont alignés avec le haut du corps et la tête du passager (140).

3. Système de protection contre les rayons UV (100) selon l'une quelconque des revendications 1 et 2, dans lequel les lampes UV (124) sont configurées pour émettre les rayons UV à une longueur d'onde désignée ou dans une plage de longueurs d'ondes étroite qui est sans danger pour les tissus humains.

4. Système de protection contre les rayons UV (100) selon la revendication 3, dans lequel la longueur d'onde désignée est de 222 nm.

5. Système de protection contre les rayons UV (100) selon l'une quelconque des revendications 1 à 4, comprenant en outre une unité de commande (170) et des capteurs (178), l'unité de commande (170) comprenant un ou plusieurs processeurs (182) et étant connectée de manière opérationnelle aux lampes UV (124) et aux capteurs (178), les capteurs (178) étant configurés pour surveiller les sièges passagers (110), l'unité de commande (170) étant configurée pour déterminer l'occupation des sièges passagers (110) sur la base des signaux de capteur reçus des capteurs (178).

6. Système de protection contre les rayons UV (100) selon la revendication 5, dans lequel, en réponse à la détermination que le passager (140) dans le siège passager (110) a quitté le siège passager (110) pendant au moins une période de temps seuil, l'unité de commande (170) est configurée pour réduire un réglage de puissance d'au moins une des lampes UV (124) qui fournissent les deux champs d'éclairage (128).

7. Système de protection contre les rayons UV (100) selon la revendication 5 ou la revendication 6, dans lequel les capteurs (178) comprennent au moins l'un des capteurs suivants : des capteurs de pression (178), des capteurs optiques (178) ou des capteurs de proximité (178).

8. Système de protection contre les rayons UV (100) selon l'une quelconque des revendications 1 à 4, comprenant en outre une unité de commande (170) et des capteurs (178), l'unité de commande (170) comprenant un ou plusieurs processeurs (182) et étant connectée de manière opérationnelle aux lampes UV (124) et aux capteurs (178), les capteurs (178) étant disposés sur ou à proximité des lampes UV (124), l'unité de commande (170) étant configurée pour détecter lorsque des personnes se trouvent à l'intérieur d'un seuil de proximité désigné des lampes UV (124) sur la base de signaux de capteur reçus des capteurs (178) ; et, en option, dans lequel, en réponse à la détection qu'une personne se trouve à l'intérieur du seuil de proximité désigné d'une première lampe UV parmi les lampes UV (124), l'unité de commande (170) est configurée pour désactiver la première lampe UV ou réduire un réglage de puissance de la première lampe UV sans désactiver la première lampe UV.

9. Système de protection contre les rayons UV (100) selon l'une quelconque des revendications 1 à 8, dans lequel le véhicule est un aéronef (10).

10. Procédé de désinfection pour protéger les passagers assis dans des sièges (110) disposés à l'intérieur d'une cabine interne d'un véhicule, chaque siège passager comprenant un dossier (114) ayant une partie avant (126), le procédé comprenant :
le montage de lampes UV (124) à l'intérieur de la cabine interne (122) du véhicule, les lampes UV (124) étant montées de manière à émettre et à diriger les rayons UV dans des champs d'éclairage (128) qui s'étendent le long des côtés (118, 120) des sièges passagers (110), deux champs d'éclairage adjacents (128) étant espacés pour définir une zone de protection (130) pour un passager (140) assis sur l'un des sièges passagers (110), la zone de protection s'étendant le long des deux côtés de l'un des sièges passagers ; et
commander les lampes UV (124) pour émettre la lumière UV dans les champs d'éclairage (128) le long des deux côtés de l'un des sièges passagers ; où deux des lampes UV (124) sont montées à l'avant (126) du dossier (114) dudit siège passager (110) de telle sorte que l'une des deux lampes UV (124) soit située au niveau ou à proximité du côté intérieur (118) du siège passager (110) et l'autre des deux lampes UV (124) soit située au niveau ou à proximité du côté extérieur (120) du siège passager (110), les deux lampes UV (124) fournissant les deux champs d'éclairage adjacents (128).

11. Procédé selon la revendication 10, dans lequel les lampes UV (124) sont commandées :
pour façonner l'émission de rayons UV de telle sorte que les champs d'éclairage (128) soient allongés dans une dimension verticale et étroits dans une dimension latérale ; et/ou
de telle sorte que les rayons UV émis aient une longueur d'onde désignée ou une plage de longueurs d'ondes étroite qui soit sans danger pour les tissus humains en cas d'exposition prolongée.

12. Procédé selon la revendication 10 ou la revendication 11, comprenant en outre la détermination :
qu'un des sièges passagers (110) est inoccupé pendant au moins une période de temps seuil et, en réponse, la réduction d'un réglage de puissance d'au moins une des lampes UV (124) qui fournissent des champs d'éclairage (128) qui s'étendent le long du siège passager inoccupé (110) ; et/ou
que l'un des passagers se trouve à une distance inférieure à un seuil de proximité désigné par rapport à l'une des lampes UV (124) et, en réponse, la réduction de la puissance de ladite lampe UV.

13. Système de protection contre les rayons ultraviolets (UV) (100) comprenant :
le système de protection contre les rayons UV (100) selon l'une quelconque des revendications 1 à 4 ;
des capteurs (178) configurés pour surveiller l'occupation des sièges passagers (110) et la proximité des passagers par rapport aux lampes UV (124) ; et
une ou plusieurs unités de commande (170) comprenant un ou plusieurs processeurs (182) et connectées de manière opérationnelle aux lampes UV (124) et aux capteurs (178), l'unité de commande (170) étant configurée pour recevoir des signaux provenant des capteurs (178) et pour moduler la puissance de sortie d'une ou plusieurs des lampes UV (124) en fonction de l'occupation des sièges passagers (110) et de la proximité des passagers par rapport aux lampes UV (124).
